(19) Europäisches Patentamt
European Patent Office
Office européen des brevets

(11) **EP 4 083 001 A1**

(12) **EUROPEAN PATENT APPLICATION**
published in accordance with Art. 153(4) EPC

(43) Date of publication:
**02.11.2022 Bulletin 2022/44**

(21) Application number: **20906893.1**

(22) Date of filing: **28.12.2020**

(51) International Patent Classification (IPC):
***C07C 29/149*** *(2006.01)*    ***C07C 35/14*** *(2006.01)*
***B01J 23/62*** *(2006.01)*

(52) Cooperative Patent Classification (CPC):
**B01J 23/62; C07C 29/149; C07C 35/14**

(86) International application number:
**PCT/KR2020/019185**

(87) International publication number:
**WO 2021/133136 (01.07.2021 Gazette 2021/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.12.2019 KR 20190176139**
**24.12.2020 KR 20200183547**

(71) Applicant: **Hanwha Solutions Corporation
Jung-gu
Seoul 04541 (KR)**

(72) Inventors:
• **JANG, Namjin**
 **Daejeon 34128 (KR)**
• **LEE, Sun Uk**
 **Daejeon 34128 (KR)**
• **KIM, Eun Jeong**
 **Daejeon 34128 (KR)**

(74) Representative: **Berggren Oy
P.O. Box 16
Eteläinen Rautatiekatu 10A
00101 Helsinki (FI)**

(54) **METHOD FOR PRODUCING 1,4-CYCLOHEXANE DICARBOXYLIC ACID**

(57)    This invention relates to a method for preparing 1,4-cyclohexane dicarboxylic acid(CHDA). More specifically, this invention relates to a method for preparing 1,4-cyclohexane dicarboxylic acid having a high rate of trans isomers, without an isomerization reaction step.

EP 4 083 001 A1

Description

## BACKGROUND OF THE INVENTION

**(a) Field of the Invention**

CROSS-REFERENCE TO RELATED APPLICATION(S)

This application claims the benefit of Korean Patent Application No. 10-2019-0176139 filed on December 27, 2019 and of Korean Patent Application No. 10-2020-0183547 filed on December 24, 2020 with the Korean Intellectual Property Office, the disclosures of which are herein incorporated by reference in their entirety.

This invention relates to a method for preparing 1,4-cyclohexane dicarboxylic acid(CHDA). More specifically, this invention relates to a method for preparing 1,4-cyclohexane dicarboxylic acid having a high rate of trans isomers, without an isomerization reaction step.

**(b) Description of the Related Art**

1,4-cyclohexanedimethanol(CHDM) is widely used as the raw material of medicine, synthetic resin, synthetic fiber or dye, and the like, and particularly, used as the raw material of environment-friendly polyethyleneterephthalate.

1,4-cyclohexanedimethanol exists as stereoisomers of cis and trans forms, and for higher quality product, it is required to have a higher rate of trans 1,4- cyclohexanedimethanol(trans CHDM) than cis CHDM.

Among the methods for preparing 1,4-cyclohexanedimethanol, a method by the hydrogenation reaction of dimethyl terephthalate(DMT) is commercially mainly used. According to this method, phthalate is reacted with methanol to prepare DMT, followed by two-step hydrogenation to prepare 1,4-cyclohexanedimethanol. The first hydrogenation reaction converts DMT into DMCD(diester dimethyl 1,4-cyclohexanedicarboxylate), and the second hydrogenation reaction converts DMCD into CHDM. Wherein, the ratio of cis CHDM and trans CHDM is determined according to the kind of a catalyst. In case a copper chromite catalyst, which is commercially mainly used copper chrome oxide, is used, the ratio of cis CHDMA and trans CHDM may become about 3:7. Since this method uses DMT and involves a trans esterification reaction using methanol, reaction and separation processes are complicated, and for isomerization, additives should be added so as to influence the quality of the final product.

According to another method, phthalate is first hydrogenated and converted into 1,4-cyclohexane dicarboxylic acid(CHDA), and CHDA is hydrogenated into CHDM. This method uses a heterogeneous catalyst and consists of two step hydrogenation reactions.

Korean Laid-Open Patent Publication No. 2015-0062911 suggested a method for preparing CHDM through two step reduction processes of terephthalic acid. However, this method has a problem of low trans CHDM rate.

Korean Registered Patent No. 0943872 suggested a method of separating intermediate trans CHDA so as to increase trans CHDM rate. According to this method, simultaneously with progressing an isomerization reaction using melting point difference between cis CHDA and trans CHDA, trans CHDA is produced as a solid or molten state. However, this method requires a process of removing water used as a solvent or other solvents, and should be operated at low temperature to remove heat used in the reduction process of phthalate for recrystallization, and thus, is not economically efficient.

As another method, Japanese Laid-Open Patent Publication No. 2014-177422 suggested a method of obtaining desired trans CHDM rate by controlling the temperature and time of hydrogenation reaction. This method progresses an isomerization reaction simultaneously with a reduction reaction of CHDA, and adopts means for controlling reaction temperature and reaction time in a fixed bed reactor, but the fixed bed reactor easily progresses crystallization during conversion of reactant CHDA, and catalyst performance decreases by crystallization, and thus, desired yield and trans CHDM rate cannot be achieved.

[Patent Document]

    (Patent Document 0001) Korean Laid-Open Patent Publication No. 2015- 0062911호

    (Patent Document 0002) Korean Registered Patent No.0943872

    (Patent Document 0003) Japanese Laid-Open Patent Publication No. 2014-177422

## SUMMARY OF THE INVENTION

In order to solve the problems of the prior art, it is an object of the invention to provide a method for preparing 1,4-cyclohexane dicarboxylic acid having high trans isomer rate, without an isomerization reaction step, by controlling the concentration of reactant terephthalic acid.

According to one aspect of the invention, there is provided a method for preparing 1,4-cyclohexane dicarboxylic acid comprising steps of:

supplying a reaction solution comprising terephthalic acid, a hydrogenation catalyst, and water to a reactor equipped with a stirrer;
supplying hydrogen gas to the reactor in which the reaction solution has been introduced; and
stirring the stirrer of the reactor to conduct a hydrogenation reaction, thus preparing 1,4-cyclohexane dicarboxylic acid(CHDA),
wherein the terephthalic acid is included in the content of 5 to 25 wt%, based on the total amount of terephthalic acid and water.

According to another aspect of the invention, there is provided a composition comprising 1,4-cyclohexane dicarboxylic acid prepared by the method.

According to the method for preparing 1,4-cyclohexane dicarboxylic acid of the invention, 1,4-cyclohexane dicarboxylic acid having high trans isomer rate can be prepared, by controlling the concentration of reactant terephthalic acid, when a hydrogenation reaction is conducted using terephthalic acid as starting material.

According to the preparation method, additional isomerization step is not conducted, and thus, the process may be simplified and economical, and prepared CHDA has high trans isomer rate, and in CHDM obtained in the subsequent hydrogenation process, such a high isomer rate may be maintained or even increase, and thus, when using it as raw material of polymer, property improvement can be expected

## DETAILED DESCRIPTION OF THE EMBODIMENTS

The terms used herein are only to explain specific embodiments, and are not intended to limit the invention. A singular expression includes a plural expression thereof, unless it is expressly stated or obvious from the context that such is not intended. As used herein, the terms "comprise" , "equipped" or "have" , etc. are intended to designate the existence of practiced characteristic, number, step, constructional element or combinations thereof, and they are not intended to preclude the possibility of existence or addition of one or more other characteristics, numbers, steps, constructional elements or combinations thereof.

Although various modifications can be made to the invention and the invention may have various forms, specific examples will be illustrated and explained in detail below. However, it should be understood that these are not intended to limit the invention to specific disclosure, and that the invention includes all the modifications, equivalents or replacements thereof without departing from the spirit and technical scope of the invention.

Hereinafter, a method for preparing 1,4-cyclohexane dicarboxylic acid according to specific embodiments of the invention will be explained in detail.

According to one embodiment of the invention, there is provided a method for preparing 1,4-cyclohexane dicarboxylic acid comprising steps of: supplying a reaction solution comprising terephthalic acid, a hydrogenation catalyst, and water to a reactor equipped with a stirrer; supplying hydrogen gas to the reactor in which the reaction solution has been introduced; and stirring the stirrer of the reactor to conduct a hydrogenation reaction, thus preparing 1,4-cyclohexane dicarboxylic acid(CHDA), wherein the terephthalic acid is included in the content of 5 to 25 wt%, based on the total amount of terephthalic acid and water.

By the hydrogenation reaction, the aromatic ring of terephthalic acid is hydrogenated, and it is converted into corresponding 1,4-cyclohexane dicarboxylic acid.

In case the hydrogenation reaction of terephthalic acid is conducted in the presence of a hydrogenation catalyst to prepare 1,4-cyclohexane dicarboxylic acid, 1,4-cyclohexane dicarboxylic acid obtained as the reaction product is in the form of a mixture of cis isomers and trans isomers, namely, cis CHDA and trans CHDA.

According to Korean Laid-Open Patent Publication No. 2020-0081096, it is disclosed that as the result of the hydrogenation reaction of terephthalic acid, a mole ratio of cis isomers and trans isomers is about 8:2 to about 6:4, thus obtaining more cis isomers, and that the mole ratio of cis isomers and trans isomers is generally within the above range regardless of the kind of the hydrogenation catalyst or detailed conditions of the hydrogenation reaction.

Thus, previously, in order to prepare 1,4-cyclohexane dicarboxylic acid having high trans content, a hydrogenation reaction of terephthalic acid into 1,4-cyclohexane dicarboxylic acid was conducted, and then, an isomerization reaction step for converting cis isomers of 1,4-cyclohexane dicarboxylic acid into trans isomers of 1,4-cyclohexane dicarboxylic acid was necessarily conducted. However, such a method is complicated and inefficient and requires additional production cost, due to the isomerization process, and thus, is not commercially preferable.

However, the method for preparing 1,4-cyclohexane dicarboxylic acid according to one embodiment of the invention can prepare 1,4-cyclohexane dicarboxylic acid having high trans isomer content only by a hydrogenation process of terephthalic acid, by controlling the concentration of reactant terephthalic acid and the concentration of a hydrogenation catalyst.

When progressing a hydrogenation reaction of a compound having cis/trans isomers, it is generally expected that cis and trans ratios of the reactant and product before and after the reaction may be maintained without significant change. Even if the concentration of the reactant is increased, only improvement in the reaction speed may be anticipated, and commonly, change in isomer ratio of the hydrogenation reaction product is not expected.

However, according to one embodiment of the invention, when the concentration of terephthalic acid is within a specific range, trans isomer content of the product 1,4-cyclohexane dicarboxylic acid unexpectedly increases without additional isomerization reaction step. It is believed that the isomerization speed varies according to the concentration of terephthalic acid, and the concentration of 1,4-cyclohexane dicarboxylic acid rapidly increases, and thus, at specific concentration of terephthalic acid, a time for thermodynamically reaching the equilibrium of trans/cis isomer ratio is shortened.

More specifically, in the preparation method of 1,4-cyclohexane dicarboxylic acid according to one embodiment of the invention, a reaction solution comprising terephthalic acid, a hydrogenation catalyst, and water is introduced in a reactor equipped with a stirrer.

The terephthalic acid is included in the content of 5 to 25 wt%, based on the total amount of terephthalic acid and water. More specifically, the content of the terephthalic acid may be 5 wt% or more, or 10 wt% or more, or 15 wt% or more, or 18 wt% or more, and 25 wt% or less, or 24 wt% or less, or 22 wt% or less, based on the total amount of terephthalic acid and water.

If the content of the terephthalic acid is less than 5 wt% based on the total amount of terephthalic acid and water, it takes a long time to reach the equilibrium of trans/cis isomer ratio, and thus, the rate of trans isomers in produced CHDA may be low, and if it is greater than 25 wt%, due to low solubility of terephthalic acid, it is difficult to dissolve, and a reaction temperature should be set high. If the reaction temperature increases, low boiling by-products may be generated in large quantities, thus decreasing yield, and catalytic activity may decrease due to thermal fatigue.

According to one embodiment of the invention, as the hydrogenation catalyst, catalysts known to be usable for a hydrogenation reaction of terephthalic acid may be used.

According to one embodiment of the invention, the hydrogenation catalyst may comprise one or more metals selected from the group consisting of palladium(Pd), rhodium(Rh), ruthenium(Ru), and platinum(Pt), as an active component.

Preferably, the hydrogenation catalyst may comprise palladium(Pd) as an active component.

According to one embodiment of the invention, the amount of the active component of the hydrogenation catalyst may be appropriately controlled according to the content of terephthalic acid. Specifically, as the content of the catalyst is higher compared to terephthalic acid, a reaction speed increases, and thus, the hydrogenation catalyst may be added in such an amount that the weight ratio of the hydrogenation catalyst and terephthalic acid may become 0.01:1 or more.

However, in case the content of the hydrogenation catalyst is above a certain level compared to terephthalic acid, the reaction speed increasing effect may be insignificant compared to the amount used, and thus, reaction efficiency may decrease. Thus, the hydrogenation catalyst may be more specifically added in an amount fulfilling the weight ratio of the hydrogenation catalyst and terephthalic acid of 0.01 : 1 to 3 : 1, or 0.01 : 1 to 2.5 : 1, or 0.1 : 1 to 2: 1.

However, the scope of the invention is not limited by the above weight ratio, and the rate of the catalyst may be appropriately controlled according to specific reaction conditions and the kind of a reactor.

The hydrogenation catalyst may be supported on a carrier, and as the carrier, those known in the art may be used without limitations. Specifically, carbon, zirconia($ZrO_2$), titania$^{\circ \vdash}$($TiO_2$), alumina($Al_2O_3$), or silica($SiO_2$), and the like may be used.

When carbon is used as the carrier, although not limited, at least one selected from the group consisting of activated carbon, carbon black, graphite, graphene, OMC (ordered mesoporous carbon) and carbon nanotube may be used.

Preferably, it may be carbon black having a high rate of mesopores in the total pores, and specifically, the activated carbon may be SXULTRA, CGSP, PK1-3, SX 1G, DRACO S51HF, CA-1, A-51, GAS 1240 PLUS, KBG, CASP and SX PLUS, and the like, and the carbon black may be BLACK PEARLS®, ELFTEX®, VULCAN®, MOGUL®, MONARCH®, EMPEROR®, and REGAL®, and the like, but not limited thereto.

Wherein, according to the invention, in the carbon carrier, the volume fraction of mesopores having sizes of 2 to 50 nm in the total pores may be 50% or more. Preferably, in the carbon carrier, the volume fraction of mesopores in the total pores may be 70% or more, and more preferably, in the carbon carrier, the volume fraction of mesopores in the total pores may be 75% or more.

Wherein, if the volume fraction of mesopores is less than 50%, there may be a problem in terms of a speed of microscopic transfer of the reactant and product in the carbon carrier, and if the average size of the pores is greater than 50 nm, the physical strength of the carrier may be weak, and thus, the above ranges are preferable.

And, according to the invention, the carbon comprises ordered mesoporous carbon(OMC) having specific surface area(BET) of 100 to 1,500 $m^2/g$. Preferably, the carbon may comprise ordered mesoporous carbon(OMC) having specific surface area(BET) of 200 to 1,000 $m^2/g$. Wherein, if the specific surface area of carbon is less than 100 $m^2/g$, it may be difficult to highly disperse active metal, and if the specific surface area of carbon is greater than 1,500 $m^2/g$, the rate of mesopores may decrease, and thus, the above ranges are preferable.

And, in some cases, the carbon carrier of the catalyst according to the invention comprises an appropriate rate of micropores, besides mesopores, and preferably, the volume fraction of micropores may be 0 to 25% in the total pores. Wherein, in case the volume fraction of micropores is greater than 25%, there may be a problem in terms of a speed of microscopic transfer of reactant and product in the carbon carrier, and thus, the above range is preferable.

In case the hydrogenation catalyst is supported on a carrier, the amount of the active component of the hydrogenation catalyst may be preferably 20 parts by weight or less, based on 100 parts by weight of the carrier, and it may be 15 parts by weight or less, or 10 parts by weight or less, and 1 part by weight or more, or 3 parts by weight or more. If the amount of the hydrogenation catalyst is too large based on 100 parts by weight of the carrier, a reaction may rapidly progress on the catalyst surface, during which side reactions may also increase and the amount of by-products may rapidly increase, and if the amount of the hydrogenation catalyst is too small, catalyst amount may be insufficient, and thus, yield of the hydrogenation reaction may decrease, and thus, the above range is preferable.

The method for preparing 1,4-cyclohexane dicarboxylic acid according to one embodiment of the invention may be conducted using a reactor comprising a stirrer, a raw material inlet, a metal sintered filter, and a product outlet.

For example, the stirrer may be a gas-induced type stirrer comprising a gas inlet, a gas passage, an impeller and jet orifices.

More specifically, the stirrer is provided in the up and down direction of the reactor, and the upper part may be provided with a gas inlet for inhaling gas, namely, hydrogen gas, by centrifugal force. The hydrogen gas inhaled in the gas inlet is passed to the lower part of the reactor through the gas passage. The hydrogen gas passed to the lower part of the reactor is sprayed and fed into the reaction solution through plural jet orifices of the stirrer, thus conducting a hydrogenation reaction. The jet orifice may be positioned at the lower part, on the side, or both at the lower part and on the side of the stirrer.

As such, as a hydrogenation reaction is conducted while spraying and mixing hydrogen gas inhaled through the gas inlet into the reaction solution through the plural jet orifices of the stirrer, hydrogenation reaction speed may increase.

And, since the stirrer comprises an impeller stirring the reaction solution, gas holdup and surface area per unit volume may increase. Thus, a hydrogenation reaction speed in the reactor may increase.

The impeller may be arranged in multi stages at the rotation axis of the stirrer.

Alternatively, according to another embodiment, only an impeller having jet orifices may be provided at the lower part of the stirrer, and additional impellers may not be provided.

The rotation axis may be operated by a driving motor equipped outside.

The lower part of the reactor may be connected to the raw material inlet, and raw materials, namely, terephthalic acid, solvents, and hydrogen gas may be introduced therein.

Meanwhile, the reactor may comprise a metal sintered filter for filtering a catalyst from the product, and a product outlet, wherein the metal sintered filter may be connected to the product outlet and installed. And, the metal sintered filter may be connected to the product outlet and provided outside of the reactor. The metal sintered filter may effectively filter catalyst components remaining in the product.

Next, in the reactor where the reaction solution has been introduced, hydrogen gas is fed.

The hydrogenation reaction may be conducted in liquid phase or gas phase. According to one embodiment of the invention, a hydrogenation reaction may be progressed while terephthalic acid is a liquid phase dissolved in a solvent such as water, and hydrogen is a gas phase,

Next, by stirring the stirrer of the reactor to conduct a hydrogenation reaction, 1,4-cyclohexane dicarboxylic acid is prepared.

Although the hydrogenation reaction conditions are not specifically limited herein, for example, a reaction temperature may be 230°C or more, and 300°C or less, or 280 °C or less, or 270°C or less. If the reaction temperature is less than 230 °C, contact with a catalyst may decrease or the temperature may not fall within a temperature at which the catalyst is activated, and thus, a reaction speed may decrease, or the content of trans isomers in produced CHDA may decrease, and if the reaction temperature is greater than 300 °C, by-products may rapidly increase, and catalyst life may be also influenced, and thus, the above range is preferable.

And, a reaction pressure may be 50 bar or more, or 80 bar or more, and 220 bar or less, or 200 bar or less, or 180 bar or less. If the reaction pressure is less than 50 bar, a reaction may not sufficiently occur, and thus, an excessive amount of catalyst may be consumed, and residence time may be too lengthened, thus causing a lot of problems such as by-product increase, and if the reaction pressure is greater than 220 bar, excessive energy may be required during process operation, and the production cost of equipment such as a reactor may significantly increase, and thus, the above range is preferable.

Since the reaction pressure is a pressure established by hydrogen gas supplied, it may be controlled according to the amount of hydrogen gas supplied.

During the hydrogenation reaction, a stirring process is conducted, and the reaction efficiency of the hydrogenation reaction may be increased through control of the stirring speed. Specifically, the stirring process may be conducted such that the surface area per unit volume of hydrogen gas bubbles may become 15 $m^2/m^3$ or more, more specifically, 50 $m^2/m^3$ or more, or 100 $m^2/m^3$ or more, or 150 $m^2/m^3$ or more, or 200 $m^2/m^3$ or more, or 300 $m^2/m^3$ or more.

As long as the surface area per unit volume meets a certain level, for example, 15 $m^2/m^3$ or more, a reaction speed is slower than a speed at which hydrogen gas is dissolved, and thus, a reaction speed may not be significantly influenced. Thus, the upper limit of the surface area per unit volume is not specifically limited as long as it meets 15 $m^2/m^3$ or more, but considering the energy efficiency of a reactor, it is preferably 500 $m^2/m^3$ or less.

Meanwhile, the stirring process may be conducted using the stirrer of the reactor as explained above.

It may be more preferable in terms of process efficiency that the reaction is conducted for 1 to 10 hours under conditions fulfilling all the hydrogenation reaction conditions as described above.

In the reaction product obtained after the reaction, CHDA comprising cis isomers and trans isomers, solvent water, and a catalyst, and the like are included, and it is used as the reactant of various reactions, or used as the reactant of the subsequent hydrogenation reaction of step 2 (hydrogenation reaction of CHDA to CHDM). It may be sent as the reactant of the hydrogenation reaction of step 2, after removing the catalyst included in the reaction product by a catalyst filter, and the like, as necessary.

According to one embodiment of the invention, the amount of 1,4-cyclohexane dicarboxylic acid comprising cis isomers and trans isomers may be 5 to 30 wt%, based on the total amount of the reaction product. More specifically, it may be 5 wt% or more, or 7 wt% or more, or 10 wt% or more, and 30 wt% or less, or 25 wt% or less, or 23 wt% or less.

According to one embodiment of the invention, in the mixed solution comprising terephthalic acid, a hydrogenation catalyst and water, the amount of terephthalic acid may be 5 to 25 wt%, preferably 10 to 25 wt%, more preferably, 12 to 22 wt%, based on the total amount of reactant terephthalic acid and water. In case the mixed solution comprising the above content of terephthalic acid is subjected to a hydrogenation reaction to prepare 1,4-cyclohexane dicarboxylic acid, in the total 1,4-cyclohexane dicarboxylic acid prepared, the rate of trans isomers may be 60 wt% or more, or 62 wt% or more, or 65 wt% or more, or 67 wt% or more, or 70 wt% or more, and although there is no upper limit of the trans isomer rate, for example, it may be 80 wt% or less, or 78 wt% or less, or 75 wt% or less.

Thus, 1,4-cyclohexane dicarboxylic acid obtained by the preparation method of the invention has high trans isomer content of 60wt% or more, and thus, can be usefully used as the raw material for preparing higher quality products, without additional isomerization process.

According to another embodiment of the invention, there is provided a composition comprising 1,4-cyclohexane dicarboxylic acid prepared by the method for preparing 1,4-cyclohexane dicarboxylic acid.

In order to immediately use 1,4-cyclohexane dicarboxylic acid in the hydrogenation reaction of step 2 (hydrogenation reaction of CHDA into CHDM) without an isomerization process, the content of trans isomers in 1,4-cyclohexane dicarboxylic acid should be 60wt% or more.

The composition comprising 1,4-cyclohexane dicarboxylic acid of the invention may have very high trans isomer rate such as 60wt% or more, or 62wt% or more, or 65wt% or more, or 67wt% or more, or 70wt% or more, in 1,4-cyclohexane dicarboxylic acid. And, although the upper limit of the trans isomer rate is not limited, for example, it may be 80wt% or less, or 78wt% or less, or 75wt% or less.

The composition of one embodiment may be used as the raw material of medicine, synthetic resin, synthetic fiber or dye.

Hereinafter, the invention will be explained in more detail through examples for better understanding of the invention. However, these examples are presented only as the illustrations of the invention, and the invention is not limited thereby.

**<Example>**

**Example 1**

A reactor equipped with a gas-induced type stirrer was prepared.

In the reactor, 286g of terephthalic acid(TPA), 92g of hydrogenation catalyst Pd/C(comprising 5wt% of Pd, based on the carrier carbon), and 2,100g of solvent distilled water were introduced as reactants, and the inner atmosphere of the reactor was replaced with nitrogen, and then, the temperature of the mixed solution was raised to 250°C while stirring at 50rpm.

After the temperature of the mixed solution reached 250°C, in order to dissolve TPA, it was stirred for 30 minutes while maintaining the temperature. And then, the stirring speed was increased, and while supplying hydrogen in the reaction solution such that the internal pressure of the reactor is maintained at 120 bar, and surface area per unit volume of hydrogen gas is maintained at 300 to 500 $m^2/m^3$, a hydrogen reaction was conducted for 1 hour.

## Example 2

The same procedure as the step 1 of Example 1 was conducted, except that 378g of terephthalic acid(TPA) was used in Example 1.

## Example 3

The same procedure as the step 1 of Example 1 was conducted, except that 492g of terephthalic acid(TPA) was used in Example 1.

## Example 4

The same procedure as the step 1 of Example 1 was conducted, except that 592g of terephthalic acid(TPA) was used in Example 1.

## Comparative Example 1

As a reactor, a batch reactor capable of withstanding at 300°C, 150 bar was prepared. Into the batch reactor, 1.5g of terephthalic acid(TPA), 1g of 5wt% hydrogenation catalyst Pd/C, and 250g of distilled water solvent were introduced as reactants, and the inner atmosphere of the reactor was replaced with nitrogen, and then, the temperature of the mixed solution was raised to 250°C while stirring at 50rpm.

After the temperature of the mixed solution reached 250°C, in order to dissolve TPA, it was stirred for 30 minutes while maintaining the temperature. And then, hydrogen was filled to the hydrogen pressure of 120 bar in the reactor, and the stirring speed was increased to 800rpm, and a hydrogenation reaction was conducted for 1 hour while stirring.

## Comparative Example 2

It was attempted to conduct a hydrogenation reaction using 800g of terephthalic acid(TPA) in Example 1, but terephthalic acid was not dissolved in the reaction solution and remained as solid, and thus, a hydrogenation reaction was not properly progressed, and the inner wall and stirrer of the reactor were stained, and thus, the reaction was not properly progressed.

For Examples and Comparative Examples, CHDA yield, conversion, and selectivity were calculated as follows, and shown in the following Table 1.

$$\mathrm{Conversion = mole\ number\ of\ reacted\ TPA\ /\ mole\ number\ of\ supplied\ TPA}$$

$$\mathrm{Selectivity = mole\ number\ of\ produced\ CHDA\ /\ mole\ number\ of\ reacted\ TPA}$$

$$\mathrm{Yield = conversion\ x\ selectivity}$$

And, trans CHDA content in the product was analyzed with gas chromatography(GC, column: HP-5, detector: FID).

[Table 1]

| | TPA content* (wt%) | Trans CHDA content in product CHDA(wt%) | TPA conversion (%) | CHDA selectivity (%) | CHDA yield (%) |
|---|---|---|---|---|---|
| Example 1 | 12 | 66 | 99.9 | 94.5 | 94.4 |
| Example 2 | 18 | 67 | 99.9 | 94.4 | 94.3 |
| Example 3 | 19 | 69 | 99.9 | 95 | 94.9 |
| Example 4 | 22 | 76 | 99.9 | 95 | 94.9 |
| Comparative Example 1 | 0.6 | 38 | 99.9 | 97 | 96.9 |
| *TPA content means the content(wt%), based on the total amount of terephthalic acid and water. | | | | | |

Referring to Table 1, it was confirmed that in the case of Examples 1 to 4 comprising a certain concentration of terephthalic acid, in the hydrogenation reaction product CHDA, trans CHDA content was higher than 66wt%, and thus, very high content of trans isomers were produced

However, in the case of Comparative Example 1 comprising 0.6wt% of terephthalic acid, in the product CHDA, only 38wt% of trans CHDA was included, and thus, sufficient trans isomers could not be produced.

**Claims**

1.  A method for preparing 1,4-cyclohexane dicarboxylic acid comprising steps of:

    supplying a reaction solution comprising terephthalic acid, a hydrogenation catalyst, and water to a reactor equipped with a stirrer;
    supplying hydrogen gas to the reactor in which the reaction solution has been introduced; and
    stirring the stirrer of the reactor to conduct a hydrogenation reaction, thus preparing 1,4-cyclohexane dicarboxylic acid(CHDA),
    wherein the terephthalic acid is included in the content of 5 to 25 wt%, based on the total amount of terephthalic acid and water.

2.  The method for preparing 1,4-cyclohexane dicarboxylic acid according to claim 1, wherein the terephthalic acid is included in the content of 12 to 22 wt%, based on the total amount of terephthalic acid and water.

3.  The method for preparing 1,4-cyclohexane dicarboxylic acid according to claim 1, wherein the step of conducting a hydrogenation reaction is conducted at a temperature of 230 to 300 °C .

4.  The method for preparing 1, 4-cyclohexane dicarboxylic acid according to claim 1, wherein the hydrogen gas is supplied at a pressure of 50 to 220 bar.

5.  The method for preparing 1, 4-cyclohexane dicarboxylic acid according to claim 1, wherein the hydrogenation catalyst comprises one or more metals selected from the group consisting of palladium(Pd), rhodium(Rh), ruthenium(Ru), and platinum(Pt).

6.  The method for preparing 1,4-cyclohexane dicarboxylic acid according to claim 1, wherein the stirring is conducted such that the surface area per unit volume of hydrogen gas bubbles becomes 15 $m^2/m^3$ or more.

7.  The method for preparing 1,4-cyclohexane dicarboxylic acid according to claim 1, wherein the 1,4-cyclohexane dicarboxylic acid comprises 60wt% or more of trans isomers.

8.  A composition comprising 1,4-cyclohexane dicarboxylic acid prepared by the method of claim 1.

## INTERNATIONAL SEARCH REPORT

| | International application No. |
|---|---|
| | **PCT/KR2020/019185** |

**A.     CLASSIFICATION OF SUBJECT MATTER**

**C07C 29/149**(2006.01)i; **C07C 35/14**(2006.01)i; **B01J 23/62**(2006.01)i

According to International Patent Classification (IPC) or to both national classification and IPC

**B.     FIELDS SEARCHED**

Minimum documentation searched (classification system followed by classification symbols)

C07C 29/149(2006.01); B01J 23/44(2006.01); C07C 29/00(2006.01); C07C 31/27(2006.01); C07C 51/36(2006.01); C07C 61/09(2006.01); C07C 67/303(2006.01); C07C 67/62(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 1,4-사이클로헥산 디카르복실산(1,4-cyclohexane dicarboxylic acid), 테레프탈산 (terephthalic acid), 수소첨가(hydrogenation), 트랜스-이성질체(trans-isomer)

**C.     DOCUMENTS CONSIDERED TO BE RELEVANT**

| Category* | Citation of document, with indication, where appropriate, of the relevant passages | Relevant to claim No. |
|---|---|---|
| X | KR 10-0272065 B1 (TOWA CHEMICAL INDUSTRY CO., LTD.) 15 November 2000 (2000-11-15) See claim 1; page 3, lines 54-58; and example 1. | 1,2,5-8 |
| Y | | 3,4 |
| Y | KR 10-2018-0035585 A (LOTTE CHEMICAL CORPORATION) 06 April 2018 (2018-04-06) See paragraph [0100]; and example 1. | 3,4 |
| X | JP 2002-069016 A (MITSUBISHI CHEMICALS CORP.) 08 March 2002 (2002-03-08) See claim 1; and paragraph [0014]. | 1,5,8 |
| A | WO 2019-046412 A1 (CLEARWATERBAY CHDM TECHNOLOGY LIMITED et al.) 07 March 2019 (2019-03-07) See entire document. | 1-8 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **09 April 2021** | **09 April 2021** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

| INTERNATIONAL SEARCH REPORT | International application No. |
|---|---|
| | **PCT/KR2020/019185** |

**C.    DOCUMENTS CONSIDERED TO BE RELEVANT**

| Category* | Citation of document, with indication, where appropriate, of the relevant passages | Relevant to claim No. |
|---|---|---|
| A | KR 10-1797220 B1 (HANWHA CHEMICAL CORPORATION) 13 November 2017 (2017-11-13)<br>See entire document. | 1-8 |

Form PCT/ISA/210 (second sheet) (July 2019)

<table>
<tr><td colspan="2" align="center"><b>INTERNATIONAL SEARCH REPORT</b><br><b>Information on patent family members</b></td><td colspan="2">International application No.<br><b>PCT/KR2020/019185</b></td></tr>
</table>

| Patent document cited in search report | Publication date (day/month/year) | Patent family member(s) | Publication date (day/month/year) |
|---|---|---|---|
| KR 10-0272065 B1 | 15 November 2000 | EP 0603825 A1 | 29 June 1994 |
| | | EP 0603825 B1 | 02 September 1998 |
| | | JP 06-184041 A | 05 July 1994 |
| | | JP 3106411 B2 | 06 November 2000 |
| | | KR 10-1994-0014291 A | 18 July 1994 |
| | | US 5430184 A | 04 July 1995 |
| KR 10-2018-0035585 A | 06 April 2018 | KR 10-1917366 B1 | 09 November 2018 |
| JP 2002-069016 A | 08 March 2002 | None | |
| WO 2019-046412 A1 | 07 March 2019 | CN 111263745 A | 09 June 2020 |
| | | KR 10-2020-0044109 A | 28 April 2020 |
| | | US 10329235 B2 | 25 June 2019 |
| | | US 2019-0062251 A1 | 28 February 2019 |
| KR 10-1797220 B1 | 13 November 2017 | KR 10-2017-0025052 A | 08 March 2017 |
| | | WO 2017-034160 A1 | 02 March 2017 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190176139
- KR 1020200183547
- KR 20150062911
- KR 0943872
- JP 2014177422 A
- KR 20200081096